# EUROPÄISCHE PATENTSCHRIFT

(11) **EP 2 654 555 B1**
(45) Veröffentlichungstag und Bekanntmachung des Hinweises auf die Patenterteilung: **20.02.2019**
(21) Anmeldenummer: 11826110.6
(22) Anmeldetag: 20.12.2011
(51) Int. Cl.: A61B 5/022

(54) **BLUTDRUCKMESSMANSCHETTE**
BLOOD PRESSURE MEASURING CUFF
BRASSARD DE TENSIOMÈTRE

(30) Priorität: 24.12.2010 DE 102010056241
(43) Veröffentlichungstag der Anmeldung: 30.10.2013
(73) Patentinhaber: Paul Hartmann AG, 89522 Heidenheim (DE)
(72) Erfinder: WEGNER, Ronny, 89233 Neu-Ulm (DE); SCHMID, Uwe, 89558 Steinenkirch (DE); MIHO, Stevan, 89522 Heidenheim (DE); HELD, Fred, 22299 Hamburg (DE)
(86) Internationale Anmeldenummer: PCT/EP2011/006423
(87) Internationale Veröffentlichungsnummer: WO 2012/084190

(56) Entgegenhaltungen:
- DE-A1-102007 037 770
- JP-A- 2 065 838
- JP-A- 2004 195 056
- JP-A- 2007 275 483
- JP-U- 62 078 904
- JP-U- 62 078 905
- US-A1- 2005 192 501
- US-A1- 2005 251 052
- US-A1- 2007 106 166
- US-A1- 2010 268 100
- PAUL HARTMANN AG ED - PAUL HARTMANN AG: "Tensoval duo control - Gebrauchsanleitung", 89522 Heidenheim, Deutschland INTERNET CITATION, Juli 2011 (2011-07), XP002673591, Gefunden im Internet: URL:http://www.tensoval.de/fileadmin/data/ de-DE/pdfs/Gebrauchsanweisung_TDC_II.pdf [gefunden am 2012-04-10]

## Beschreibung

Die vorliegende Erfindung betrifft eine bandförmige Manschette für das Messen des Blutdrucks am Oberarm eines Menschen.

Derartige Manschetten weisen überlicherweise ein Schlaufenelement und einen Endabschnitt auf, der durch das Schlaufenelement hindurchführbar ist. Das Schlaufenelement wird dabei vorzugsweise durch einen Bügel, eine Lasche oder einen zur Hindurchführung des Endabschnitts geeigneten Schlitz gebildet. Bandförmige Manschetten dieser Bauart weisen dabei einen ersten Längsabschnitt auf, der das Schlaufenelement umfasst, wobei der erste Längsabschnitt einen ersten unteren Randbereich sowie einen ersten oberen Randbereich umfasst.

Derartige bandförmige Manschetten umfassen außerdem eine expandierbare Blase, die vorzugsweise über einen Druckschlauch mit einem Gerät zum Einleiten eines Fluids in die Blase verbindbar ist. Für eine zuverlässige Messung des Blutdrucks ist es besonders wichtig, dass die Blase im angelegten Zustand der Manschette den Oberarmbereich des Benutzers derart umschließt, dass beim Einleiten des Fluids eine Expansion der Blase die im Bereich des am Bizeps befindlichen Blutgefäße (Arteria brachialis) zuverlässig komprimiert. Da diese korrekte Positionierung der Blase für den Benutzer während des Anlegens der Manschette nicht einfach zu erkennen ist, ist bei zahlreichen im Markt befindlichen Manschetten der Druckschlauch bevorzugt auf der Außenseite des ersten Längsabschnitts, insbesondere bevorzugt im unteren Bereich des ersten Längsabschnitts in der Nähe des Schlaufenelements angeordnet. Auf diese Weise ist der Druckschlauch im angelegten Zustand der Manschette auf dem Bizeps des Benutzers oberhalb der Ellenbeuge positioniert. Diese Anordnung des Druckschlauchs zeigt dem Benutzer an, dass die Blase den entsprechenden Teil des Oberarms im Bereich des Bizeps derart umschließt, dass beim Einleiten des Fluids eine Expansion der Blase die am Bizeps befindliche Arteria brachialis zuverlässig abdrückt. Manschetten dieses Typs sind seit langem bekannt. Dem Stand der Technik entsprechende Messmanschetten sind z.B. in den japanischen Gebrauchsmuster- bzw. Patentanmeldung-Veröffentlichungen JP62078905U (20.05.1987) und JP2007275483A (25.10.2007) beschrieben.

Für eine korrekte Messung des menschlichen Blutdrucks eines Benutzers ist es essentiell, dass die Manschette, insbesondere deren Blase korrekt am Oberarm des Benutzers positioniert ist. Da ein Benutzer während des Anlegens der Manschette an dem Endabschnitt der Manschette zieht, um den Umfang der Manschette an den Umfang des Oberarms anzupassen, kommt es häufig vor, dass die Manschette in Längsrichtung, welche der Umfangsrichtung der an den Benutzer angelegten Manschette entspricht, auf dem Oberarm des Benutzers verrutscht. Dies hat eine unvorteilhafte Positionierung der Blase zur Folge, was zu einer ungenauen Messung des Blutdrucks führt. Der Benutzer ist daher gezwungen, die Position der Manschette auf dem Oberarm nachzukorrigieren. Diese umständliche Korrektur durch den Benutzer der Manschette kann zwar das Verrutschen der Manschette beim Anlegen ausgleichen, ist jedoch, insbesondere bei Anwendung der Manschette durch nicht dafür geschulte Personen, oft ungenau. Dieses Problem der genauen und einfach handhabbaren Positionierung der Manschette auf dem Oberarm des Benutzers stellt bei derzeitigem Stand der Technik ein ungelöstes Problem dar.

Der vorliegenden Erfindung liegt daher die Aufgabe zugrunde, eine Manschette für das Messen des Blutdrucks anzugeben, die die genannten Nachteile des Standes der Technik vermeidet und letzteren in vorteilhafter Weise weiterentwickelt. Insbesondere soll das Anlegen und die Positionierung der Manschette vereinfacht werden.

Zur Lösung dieser Aufgabe schlägt die Erfindung eine Manschette für das Messen des Blutdrucks gemäß Anspruch 1 vor. Dabei umfasst die Manschette an einem ersten unteren Randbereich eines ersten Längsabschnitts eine Positionierhilfe, wobei die Positionierhilfe eine Aussparung aufweist. Als ein unterer Randbereich wird im Rahmen dieser Erfindung derjenige Randbereich der Manschette verstanden, der im angelegten Zustand der Manschette in Richtung Ellenbeuge weist. Ein oberer Randbereich ist nach diesem Verständnis ein Randbereich, der in Richtung der Schulter weist. Diese Positionierhilfe ist dadurch vorteilhaft, dass die Aussparung so am Innenbereich der Ellenbeuge des Benutzers anlegbar ist, dass die Gefahr eines Verrutschens der Manschette auf dem Arm in Längsrichtung vermindert wird. Auf diese Weise ist die Blase in vorteilhafter Weise so positioniert, dass eine genaue und verlässlich reproduzierbare Messung des Blutdrucks gewährleistet ist. Der Begriff "Blase" bezeichnet dabei jede Art von Hohlkörper, der einen oder mehrere Hohlräume oder eine schwammartige Struktur aufweist und dafür geeignet ist, bei Befüllung mit einem Fluid zu expandieren. Die Blase ist in einem Blasenbereich der Manschette angeordnet, wobei sich der Blasenbereich in Längsrichtung vorzugsweise in etwa ausgehend von dem Schlaufenelement bis zu dem Ende des ersten Längsabschnitts erstreckt.

Die Blase kann von einer die Innenseite (im angelegten Zustand die körperzugewandte Seite) und einer die Außenseite (im angelegten Zustand die körperabgewandte Seite) der bandförmigen Manschette bildenden Materiallagen selbst gebildet werden, wobei diese Materiallagen solchenfalls bereichsweise, das heißt dort, wo die Materiallagen die Blase bilden, zugleich den Blasenbereich bilden und begrenzen. Die Blase kann auch in einer alternativen Ausführungsform ein separates Bauteil darstellen, das zwischen diesen Materiallagen angeordnet und dort aufgenommen ist. Denkbar und vorteilhaft wäre es nach einer weiteren Ausführungsform, wenn die Blase, vorzugsweise allseitig, von einer separaten Komponente, insbesondere von einer Hülle oder Tasche umgeben oder aufgenommen ist, die ihrerseits zwischen eine die Innenseite und eine die Außenseite der bandförmigen Manschette bildenden Materiallagen vorgesehen ist.

Die Blase hat vorzugsweise eine Länge in Längsrichtung von mindestens ca. 80% und maximal 100% des Oberarmumfangs eines Benutzers. Vorzugsweise beträgt die Länge der Blase, also deren maximale Erstreckung in der Längsrichtung, 10 cm bis 40 cm, insbesondere 22 cm bis 25 cm. Des Weiteren ist es bevorzugt, wenn die Breite der Blase, also deren maximale Erstreckung in der Querrichtung 8 cm bis 16 cm, insbesondere 11,5 cm bis 13,5 cm beträgt. Die hier angegebenen Abmessungen sind besonders für Manschetten in mittlerer und großer Größe geeignet. Denkbar und vorteilhaft ist es aber auch weitere, entsprechend angepasste Blasenabmessungen für noch kleinere oder noch größere Varianten von Manschetten vorzusehen.

Vorzugsweise weist die Manschette in an sich üblicher Weise einen Druckschlauch auf oder die Manschette ist an einen Druckschlauch anschließbar, welcher mit einem Gerät zum Einleiten eines Fluids in die Blase verbindbar ist. Ein Druckschlauch ist eine Struktur aus einer oder mehreren Leitungen, die dafür geeignet ist, ein Fluid, wie ein Gase oder eine Flüssigkeit unter Druck zu leiten und der Blase zum Zwecke der Expansion der Blase zuzuführen.

Der die Blase enthaltende Blasenbereich weist sich vorzugsweise in der Längsrichtung erstreckende untere und obere Blasenbereichsränder sowie Blasenbereichsquerränder auf.

Vorzugsweise erstreckt sich der erste Längsabschnitt über eine Länge L1 ausgehend von einem ersten Manschettenquerrand bis zu einem endabschnittnahen Blasenbereichsquerrand. Der Endabschnitt bezeichnet denjenigen Abschnitt, der sich in Längsrichtung ausgehend vom endabschnittnahen Blasenbereichsquerrand, also an den ersten Längsabschnitt anschließend, bis zu einem zweiten Manschettenquerrand erstreckt. Vorzugsweise beträgt die Länge L1 des ersten Längsabschnitts, also dessen Erstreckung in der Längsrichtung 10 cm bis 40 cm, insbesondere 20 cm bis 30 cm, weiter insbesondere 22 cm bis 25 cm.

Der sich an den ersten Längsabschnitt in der Längsrichtung anschließende Endabschnitt weist vorzugsweise keine expandierbare Blase auf. Die Länge des Endabschnitts beträgt vorzugsweise 10 cm bis 35 cm, insbesondere 24 cm bis 28 cm.

Der untere Blasenbereichsrand und die Aussparung weisen in Querrichtung, welche senkrecht zu der Längsrichtung verläuft, einen ersten Abstand von 1,5 bis 5,5 cm, vorzugsweise von 2,5 bis 4,0 cm und insbesondere von höchsten 3,0 cm voneinander auf. Diese Ausführungsform der Erfindung ist besonders vorteilhaft, weil sie im angelegten Zustand der Manschette einen geringen Abstand zwischen Blase und Ellenbeuge des Benutzers gewährleistet und so ein Verrutschen der Manschette in die Ellenbeuge hinein verhindert. Würde die Blase der angelegten Manschette in dem Bereich der Ellenbeuge mit einem Fluid befüllt werden, so würde dies zu einer ungenauen Messung des Blutdrucks führen. Diese Ausführungsform erlaubt durch den ersten Abstand eine vorteilhafte Positionierung der Manschette in Querrichtung. Der Begriff "Abstand", bezogen auf die Querrichtung, bezeichnet in dem Rahmen dieser Erfindung dabei stets die Entfernung zwischen zwei in Längsrichtung gezogenen, parallelen Hilfslinien durch die beiden Punkte, deren Abstand gemessen werden soll.

In einer bevorzugten Ausführungsform weist die Manschette eine rechteckige oder trapezförmige Kontur der Aussparung auf. In einer besonders bevorzugten Ausführungsform weist die Manschette eine im Wesentlichen bogen- oder kurvenförmige Kontur der Aussparung auf. Weitere Konturen der Aussparung, insbesondere Kombinationen aus kurvenförmigen und geradlinigen Konturabschnitten sind ebenfalls denkbar und vorteilhaft.

Es umfasst die Positionierhilfe einen ersten Flügel und einen zweiten Flügel, wobei die Aussparung in Längsrichtung beidseitig durch die Flügel begrenzt ist. Dies ist für den Benutzer von besonderem Vorteil, da die Flügel im angelegten Zustand der Manschette, in dem die Aussparung an der Ellenbeuge des Benutzers anliegt, über den Außenseiten des Ellenbogengelenks des Benutzers zu liegen kommen und die korrekte Position der Manschette zusätzlich stabilisieren. Die Flügel werden dadurch gebildet, dass der erste Längsabschnitt im Bereich der Positionierhilfe sich vorzugsweise weiter in Querrichtung nach außen erstreckt als ein Bereich außerhalb der Positionierhilfe, so dass der Manschette im Bereich der Positionierhilfe eine höhere Breite aufweist als ein der Positionierhilfe benachbarter Bereich der Manschette, insbesondere als der Endabschnitt. Die Breite B1 des ersten Längsabschnitts im Bereich der Positionierhilfe beträgt insbesondere bevorzugt 11 cm bis 24 cm, weiter insbesondere 14 cm bis 20 cm.

Besonders bevorzugt ist es hierbei, wenn die Positionierhilfe in Längsrichtung eine Länge von 10 cm bis 25 cm, noch mehr bevorzugt von 14 cm bis 18 cm aufweist.

Ebenfalls besonders vorteilhaft ist es, wenn sich die Aussparung der Positionierhilfe in Querrichtung vom distalen Ende der Flügel bis zur in Querrichtung weitesten Erstreckung der Aussparung nach innen über eine Breite von wenigstens 2 cm und höchstens 7 cm, noch bevorzugter von wenigstens 3 cm und höchstens 5 cm erstreckt.

In einer anderen bevorzugten Weiterbildung der Erfindung weist die Positionierhilfe, vorzugsweise zumindest der erste und/oder der zweite Flügel, ein Versteifungselement auf oder wird davon gebildet, wobei das Versteifungselement die Positionierhilfe, insbesondere die Form der Flügel gegen Verknicken und Verbiegen stabilisiert. Dem Benutzer wird auf diese Weise beim Anlegen die korrekte Positionierung der Manschette erleichtert. Das Versteifungselement ist dabei bevorzugt auf der Innenseite oder der Außenseite oder zwischen der die Innenseite und der die Außenseite der Positionierhilfe bildenden Materiallagen angeordnet. Des Weiteren ist es von Vorteil, wenn das Versteifungselement mindestens ein Metall oder mindestens einen Kunststoff beinhaltet oder ein mehrschichtiges Laminat umfasst. Vorteilhaft weist das Versteifungselement eine höhere Steifheit auf, als die die Positionierhilfe im Übrigen bildenden, insbesondere die die Innenseite und die Außenseite bildenden Materiallagen.

Im Falle, dass die Positionierhilfe aus dem Versteifungselement gebildet wird, weist die Positionierhilfe eine höhere Steifigkeit auf als der erste Längsabschnitt in einem Bereich außerhalb der Positionierhilfe.

Das Versteifungselement weist in Querrichtung vorzugsweise einen zweiten Abstand von höchstens 6,0 cm vorzugsweise von höchsten 5,0 cm, insbesondere zwischen 0,25 cm und 4,0 cm, weiter insbesondere zwischen 0,8 cm und 1,5 cm von dem unteren Blasenbereichsrand auf.

Nach einer alternativen Ausführungsform erstreckt sich das Versteifungselement in Querrichtung über den unteren Blasenbereichsrand, vorzugsweise um mindestens 0,5 cm, insbesondere um mindestens 3,0 cm, weiter vorzugsweise um 1,5 cm bis 10 cm hinaus. Besonders bevorzugt erstreckt sich das Versteifungselement in Querrichtung so weit über den unteren Blasenbereichsrand hinaus, dass das Versteifungselement zumindest bereichsweise die gesamte Breite der Blase überfängt.

In einer Weiterbildung des erfindungsgemäßen Gedankens einer leicht anzulegenden Manschette umfasst diese in Längsrichtung zumindest in einem ersten oberen Randbereich einen Orientierungsbereich, der sich insbesondere durch Farbe, Material oder Steifigkeit von einem in Querrichtung an den Orientierungsbereich angrenzenden Bereich der Manschette unterscheidet. Dieser Orientierungsbereich zeigt dem Benutzer an, dass jener obere Randbereich beim Anlegen der Manschette in Richtung des Schultergelenks des Benutzers angeordnet werden sollen. Es wird vermieden, dass die Manschette verkehrt herum angelegt wird und so durch eine unvorteilhafte Positionierung der Blase auf dem Bizeps (bzw. der Arteria brachialis) des Benutzers ungenaue Messwerte für den Blutdruck ermittelt werden.

Um die Manschette anzulegen, muss ein Benutzer zunächst den Endabschnitt durch das Schlaufenelement führen. Dadurch bildet die Manschette aus im Wesentlichem dem ersten Längsabschnitt einen Körper in Form eines Zylindermantels aus, wobei sich im Inneren des Zylindermantels ein Hohlraum befindet. Dieser Hohlraum kann den Oberarm des Benutzers aufnehmen. Der Innenumfang des Zylindermantels bzw. der angelegten Manschette wird dann durch Zug an dem Endabschnitt auf den Umfang des Oberarms des Benutzers angepasst. In einer anderen bevorzugten Weiterbildung des erfindungsgemäßen Gedankens einer leicht anzulegenden Manschette umfasst diese daher eine zweite Anlegehilfe, die an einem Randbereich des Endabschnitts angeordnet ist. Die zweite Anlegehilfe weist einen zweiten Anfassbereich auf, der sich in Farbe, Material oder Steifigkeit unterscheidet von einem Teilbereich des Endabschnitts, welches sich in Längsrichtung an den zweiten Anfassbereich anschließt. Dieser zweite Anfassbereich ist daher für den Benutzer leicht zu erkennen und ermöglicht es, die Manschette im angelegten Zustand an dem Endabschnitt zu greifen und durch Zug zusammenzuziehen, vorzugsweise bis der innere Umfang der Manschette dem Umfang des Oberarms des Benutzers entspricht.

In einer weiteren bevorzugten Ausführungsform umfasst die Manschette einen Druckschlauch, an dem vorzugsweise ein insbesondere mindestens zweifarbiger Informationsträger angebracht ist. Dieser Informationsträger ist Träger von Informationen vermittelnden Kennzeichnungen, welche insbesondere auf den Informationsträger aufgedruckt sein können, insbesondere in Form von Piktogrammen oder ähnlichen Druckbildern. Die Informationen vermittelnden Kennzeichnungen veranschaulichen vorzugsweise das korrekte Anlegen der Manschette durch den Benutzer, vorzugsweise mit Bezug zu den beschriebenen Positionier-, Anlege- und Orientierungshilfen.

Derartige insbesondere zwei- oder mehrfarbige Informationsträger sind nach einem weiteren Erfindungsgedanken auch auf der Außenseite der Manschette angeordnet, insbesondere auf dem in angelegten Zustand auf dem Bizeps und Trizeps des Benutzers aufliegenden und damit gut sichtbaren ersten Längsabschnitt.

In einer weiteren besonders bevorzugten Ausführungsform umfasst die Manschette zumindest an dem ersten Längsabschnitt einen sich in der Längsrichtung erstreckenden Ablesebereich mit Ablesemarkierungen. Die Ablesemarkierungen können dabei insbesondere Linien, Punkte, Symbole, Farbmuster, Zahlen, Buchstabenfolgen oder Kombinationen aus den genannten Markierungen enthalten. Außerdem ist es von Vorteil, wenn die Ablesemarkierungen dabei bevorzugt auf der Außenseite, insbesondere an einem ersten oberen Randbereich angeordnet sind. Zieht nun der Benutzer beim Anlegen der Manschette an dem Endabschnitt, um den inneren Umfang der Manschette an den Umfang des Oberarms des Benutzers anzupassen, und schließt die Manschette dann durch Auflegen eines Haftbereichs des Endabschnitts auf einen Haftbereich des ersten Längsabschnitts, so zeigt die Position des Endabschnitts im Ablesebereich an, wie weit die Manschette zugezogen wurde. Dies ermöglicht es einem Benutzer, die individuell ermittelte Ablesemarkierung, die die Position des Endabschnitts passgenau anzeigt, zu dokumentieren oder zu erinnern. Bei der nächsten Messung des Blutdrucks dienen die Ablesemarkierungen dann dazu, die Manschette erneut auf denselben Umfang einzustellen. Auf diese Weise wird die Genauigkeit und Reproduzierbarkeit von Blutdruckmessungen, insbesondere durch nicht geschulte Personen, verbessert.

Besonders vorteilhaft ist es, wenn der Ablesebereich den Orientierungsbereich bildet oder mit diesem zusammenfällt. Die Ablesemarkierungen können dann in einer weiteren bevorzugten Ausführungsform der Erfindung Zahlen sein, die auf einem andersfarbigen Streifen an dem ersten oberen Rand des ersten Längsabschnitts angeordnet sind.

In einer Weiterbildung der Erfindung ist die Positionierhilfe an einer von der Manschette ablösbaren und wieder festlegbaren Positioniervorrichtung angeordnet. Die Positioniervorrichtung umfasst vorzugsweise ein Material mit höherer Steifigkeit als das die Manschette im Bereich des ersten Längsabschnitts im Übrigen bildende Material. Insbesondere umfasst die Positioniervorrichtung ein flaches Kunststoffmaterial.

Eine mit der Positioniervorrichtung abnehmbare Positionierhilfe gestattet es, die Manschette bei Benutzern anzuwenden, welche die Positionierhilfe nicht einsetzen möchten. Auch zum Zwecke der Verpackung und Aufbewahrung ist es vorteilhaft, die Positionierhilfe von der Manschette trennen und wiederholt anbringen zu können.

Die Positioniervorrichtung weist hierzu vorzugsweise erste Befestigungsmittel auf, vermittels derer die Positioniervorrichtung auf der Manschette festlegbar ist. Vorzugsweise umfassen die ersten Befestigungsmittel an einem unteren Längsrand der Positioniervorrichtung angeordnete und sich in Querrichtung über diesen gleichsam halbinselartig hinaus erstreckende Vorsprünge, insbesondere zwei oder mehreren Vorsprünge.

Die bandförmige Manschette weist solchenfalls vorzugsweise Taschen auf, wobei die Taschen auf ihrer dem ersten unteren Randbereich der Manschette zugewandten unteren Seite offen sind. Die Taschen können auf die Außenseite der Manschette aufgesetzt sein; denkbar und vorteilhaft wäre auch, die Taschen sandwichartig zwischen die beiden Materiallagen anzuordnen, welche die Innenseite und die Außenseite der Manschette bilden. Die Verwendung von die Vorsprünge aufnehmenden Laschen anstelle von Taschen wäre ebenfalls denkbar und vorteilhaft.

Die erste Befestigungsmittel bildenden Vorsprünge sind dann zur Fixierung der Positioniervorrichtung an der Manschette in die dafür vorgesehenen Taschen einschiebbar. Vorteilhafterweise umfassen die Vorsprünge und die Taschen miteinander kooperierende Rastmittel, wie Einrastnasen und korrespondierende Haltemittel, so dass die Vorsprünge in den Taschen in einer vorgegebenen Stellung gehalten sind.

Denkbar und vorteilhaft wäre auch, das lösbare Festlegen der Positioniervorrichtung an der Manschette alternativ zu den ersten Befestigungsmitteln oder in Ergänzung dazu durch auf der Innenseite der Positioniervorrichtung vorzusehende Haftbereiche, insbesondere Klettbereiche, vorzugsweise in Form von Kletthaken, zu ermöglichen, welche mit Haftbereichen, insbesondere Klettbereichen, vorzugsweise einem Flauschmaterial, der Außenseite des ersten Längsabschnitts in Eingriff bringbar sind. In einer weiteren besonders bevorzugten Ausführungsform umfasst die Innenseite des ersten Längsabschnitts ein Haftmaterial oder wird davon gebildet, wobei dieses Haftmaterial der Innenseite des ersten Längsabschnitts auf der Haut eines Benutzers eine höhere Haftung als eine Außenseite des ersten Längsabschnitts verleiht. Diese Ausführungsform ist von Vorteil, weil sie einem Verrutschen der Manschette in Längsrichtung auf dem Oberarm des Benutzers entgegen wirkt.

Vorzugsweise umfasst der erste obere Randbereich des ersten Längsabschnitts der Manschette zum Anlegen an den Oberarm eines Menschen eine erste Anlegehilfe, die ein erstes Anfassmittel aufweist.

Vorzugsweise ist das erste Anfassmittel insbesondere durch seine Farbe, Material oder Steifigkeit von einem ersten unteren Randbereich des ersten Längsabschnitts unterscheidbar und dadurch für den Benutzer leicht zu identifizieren.

Das erste Anfassmittel ist vorzugsweise in Form einer Lasche, eines Griffs, eines Hakens, eines Henkels oder eines Bügels ausgebildet. Das erste Anfassmittel ermöglicht es dem Benutzer, beim Anlegen der Manschette die Manschette an den Oberarm eines Menschen nach oben, also in Richtung des Schultergelenks des Benutzers zu ziehen, um eine passgenauere Positionierung in Querrichtung zu gewährleisten.

In einer Weiterbildung umfasst das erste Anfassmittel einen Greifbereich und einen Markierungsbereich, wobei der Markierungsbereich eine vom Greifbereich durch Farbe, Material oder Steifigkeit, insbesondere visuell unterscheidbare Greifmarkierung aufweist, an der der Benutzer das erste Anfassmittel zwischen Zeigefinger und Daumen greifen kann.

Nachstehend werden Ausführungsformen erfindungsgemäßer bandförmiger Manschette mit Positionierhilfe anhand von Zeichnungen näher erläutert. Die Erfindung soll jedoch nicht auf die in den Zeichnungen oder in der Beschreibung der Zeichnung dargestellten Ausgestaltungen reduziert verstanden werden. Es zeigen:
**Fig. 1a und 1b** Draufsichten auf erfindungsgemäße bandförmige Manschetten für das Messen des Blutdrucks.
**Fig. 2a****)** Draufsicht auf die erfindungsgemäße bandförmige Manschette nach Figur 1a mit Darstellung weiterer Details der Manschette,
**Fig. 2b****)** Querschnitt, schematisch, durch die bandförmige Manschette entlang der Achse A-A aus Figur 2a.
**Fig. 3****)** Schematische Darstellung verschiedener Konturen von Positionierhilfen erfindungsgenmäßer Manschetten.
**Fig. 4a****)** Detailansicht einer erfindungsgemäßen Manschette mit einem Versteifungselement.
**Fig. 4b****)** Querschnitt, schematisch, durch die bandförmige Manschette entlang der Achse B-B aus Figur 4a.
**Fig. 5****)** Querschnitt analog Fig. 4b, schematisch, durch eine weitere bandförmige Manschette mit Versteifungselement.
**Fig. 6a****-d)** Detailansicht einer bandförmigen Manschette mit Anlege- und Orientierungshilfen.
**Fig. 7****)** Schematische Darstellung des Anlegevorgang einer Manschette mit erster Anlegehilfe
**Figuren 8a****·c)** Draufsicht auf eine Positioniervorrichtung, und je eine Teildraufsicht auf eine bandförmigen Manschette vor und nach dem Festlegen der Positioniervorrichtung an der Manschette

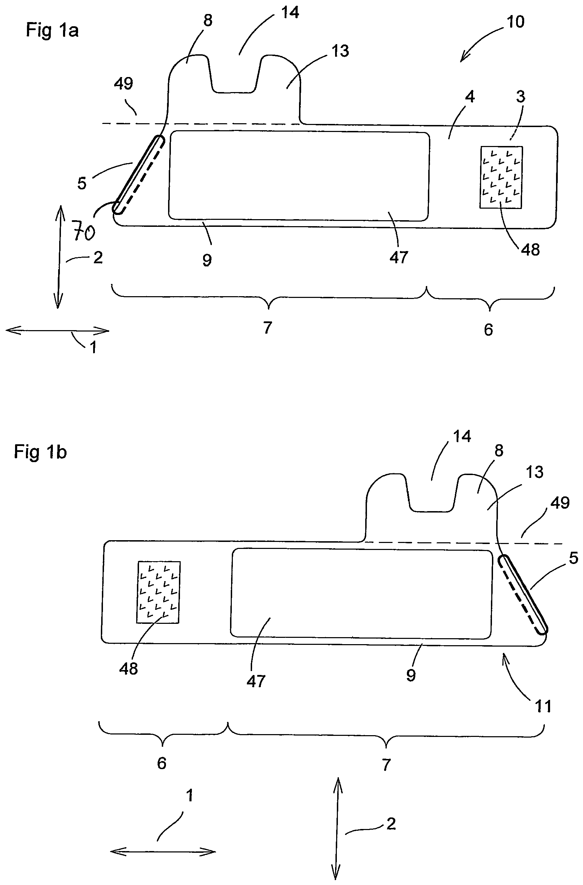

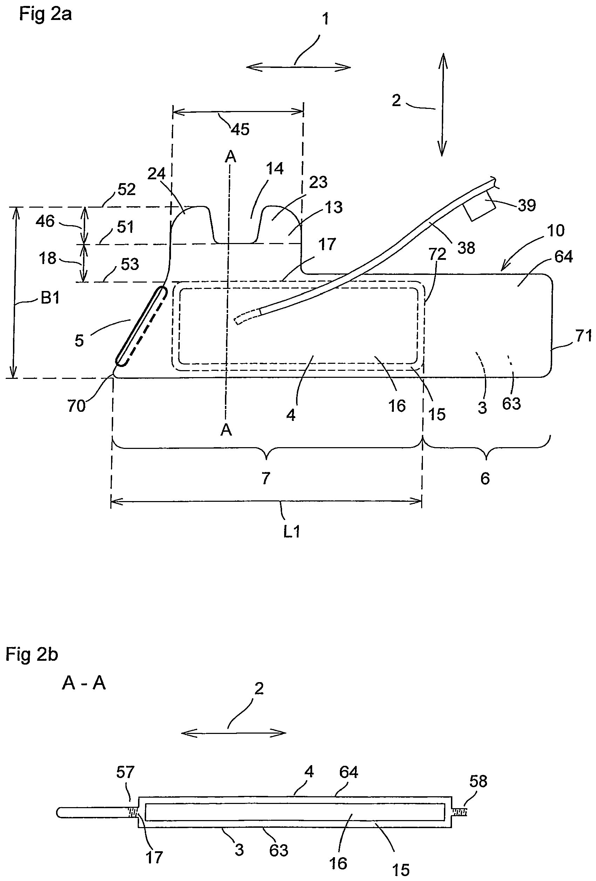

Figur 1a zeigt eine erfindungsgemäße bandförmige Manschette 10 für das Messen des Blutdrucks mit einer Längsrichtung 1 und einer Querrichtung 2, wobei die bandförmige Manschette 10 ein Schlaufenelement 5 umfasst, und mit einem Endabschnitt 6, der durch das Schlaufenelement 5 hindurch führbar ist. Das Schlaufenelement 5 ist in dieser Ausführungsart unmittelbar an einem ersten Manschettenquerrand 70 der Manschette 10 angeordnet, welcher der Positionierhilfe benachbart ist, und wird gebildet durch einen ringförmigen, metallenen Bügel, welcher einer abgerundeten Rechteckform angenähert ist und dessen eine lange Seite in einer Tasche (nicht näher dargestellt) auf der Außenseite 4 der Manschette 10 angeordnet ist, während die andere lange Seite des Bügels nicht mit der Manschette verbunden ist und so einen länglichen Schlitz für das Hindurchführen des Endabschnitts 6 darstellt. Die bandförmige Manschette 10 weist einen ersten Längsabschnitt 7 auf, der das Schlaufenelement 5 umfasst, wobei der erste Längsabschnitt 7 einen ersten unteren Randbereich 8 sowie einen ersten oberen Randbereich 9 aufweist. Beim Anlegevorgang wird der Endabschnitt 6 in an sich üblicher Weise durch das Schlaufenelement 5 hindurch geführt. Anschließend zieht der Benutzer am Endabschnitt 6 der Manschette, um den Umfang der Manschette an den Umfang des Oberarms anzupassen. Danach wird der benötigte Manschettenumfang einer Schlinge gleich durch Zurückschlagen und Anlegen der Außenseite 4 des Endabschnitts 6 auf die Außenseite 4 des ersten Längsabschnitts 7 fixiert, wobei der Endabschnitt 6 und der erste Längsabschnitt 7 auf der Außenseite 4 jeweils Haftbereiche aufweisen. Diese Haftbereiche sind in der dargestellten bevorzugten Ausführung der Manschette durch erste und zweite miteinander in Eingriff bringbare Klettbereiche gebildet. Ein erster Klettbereich ist vorzugsweise in Form einer schlaufenbildenden Zone 47 gebildet, Ein zweiter Klettbereich ist solchenfalls in Form einer Kletthaken aufweisenden Zone 48 gebildet. Im angelegten Zustand der Manschette umfasst der erste Längsabschnitt 7 im Wesentlichen jenen Bereich der Manschette, der bei korrekter Positionierung der Manschette im angelegten Zustand auf dem Bizeps und dem Trizeps des Benutzers zu liegen kommt. Vorzugsweise erstreckt sich der erste Längsabschnitt 7 wie in Figur 2a dargestellt über eine Länge L1 ausgehend von einem ersten Manschettenquerrand 70 bis zu einem endabschnittnahen Blasenbereichsquerrand 72 (Blase und Blasenbereich in Figur 1a, 1b nicht dargestellt). Der Endabschnitt 6 bezeichnet denjenigen Abschnitt, der sich in Längsrichtung 1 ausgehend vom endabschnittnahen Blasenbereichsquerrand 72, also an den ersten Längsabschnitt 7 anschließend, bis zu dem zweiten Manschettenquerrand 71 erstreckt. Die sich in der Längsrichtung 1 aneinander anschließenden Abschnitte (erster Längsabschnitt 7, Endabschnitt 6) sind vorzugsweise integral aus einem einzigen, vorzugsweise mehrlagigen Materialabschnitt gebildet. Es wäre aber auch denkbar, dass die Abschnitte aus unterschiedlichen Materialabschnitten gebildet sind, die zur Bildung der bandförmigen Manschette aneinander gefügt sind.

Der erste Längsabschnitt 7 umfasst an einem ersten unteren Randbereich 8 eine Positionierhilfe 13, wobei die Positionierhilfe 13 eine Aussparung 14 aufweist. Das den Längsabschnitt bildende Material ist also an dem unteren Randbereich 8, welcher im angelegten Zustand dem Unterarm zugewandt ist, zur Bildung einer Positionierhilfe ausgespart. Durch die Aussparung 14 kann die Manschette 10 so am Innenbereich der Ellenbeuge des Benutzers angelegt werden, dass die Gefahr eines Verrutschens der Manschette 10 auf dem Arm in Längsrichtung 1 (also im angelegten Zustand in Umfangsrichtung) vermindert wird. Auf diese Weise ist die Manschette, insbesondere die Blase 16 (siehe Figur 2a), in vorteilhafter Weise so positioniert, dass eine genaue und verlässlich reproduzierbare Messung des Blutdrucks gewährleistet ist.

Die Positionierhilfe 13 ist in dieser Variante durch eine Weitererstreckung des ersten Längsabschnitts 7 in einem dem Schlaufenelement 5 benachbarten Bereich in Querrichtung 2 über eine erste gedachte Linie 49 hinaus gebildet, wobei die gedachte Linie 49 im Wesentlichen entlang der Kontur des unteren Randes des Endabschnitts 6 verläuft. Der erste Längsabschnitt 7 erstreckt sich damit im Bereich der Positionierhilfe 13 weiter in Querrichtung 2 nach außen, also nach unten (im angelegten Zustand in Richtung auf die Ellenbeuge), als ein Bereich außerhalb der Positionierhilfe 13, so dass der Manschette 10 im Bereich der Positionierhilfe 13 eine höhere Breite B1 aufweist als ein der Positionierhilfe 13 in Längsrichtung 1 benachbarter Bereich des ersten Längsabschnitts 7 der Manschette 10, und/oder als der Endabschnitt 6.

Die Breite B1 des ersten Längsabschnitts 7 im Bereich der Positionierhilfe 13 beträgt bevorzugt 11 cm bis 24 cm, insbesondere 14 cm bis 20 cm.

Die Aussparung 14 hat im dargestellten Fall eine im Wesentlichen bogenförmige Kontur.

Figur 1a zeigt eine bandförmige Manschette 10 mit einem Schlaufenelement 5 für das Schließen der Manschette 10 auf der linken Seite, was besonders für ein Anlegen der Manschette auf dem linken Arm des Benutzers geeignet ist. Figur 1b zeigt eine bandförmige Manschette 11 mit einem Schlaufenelement 5 für das Schließen der Manschette 11 auf der rechten Seite, was besonders für ein Anlegen der Manschette auf dem rechten Arm des Benutzers geeignet ist. Im Übrigen sind die in Figuren 1a und 1b dargestellten Manschetten baugleich. Gleiche Bezugszeichen kennzeichnen in Figur 1b dieselben im Zusammenhang mit Figur 1a beschriebenen Komponenten der Manschette.

Im Folgenden werden Ausführungsformen nur der linksseitigen Manschette 10 dargestellt und beschrieben. Sämtliche im Zusammenhang mit der linksseitigen Manschette 10 beschriebenen Merkmale und Varianten sind auch im Zusammenhang mit der Ausführung rechtsseitiger Manschetten 11 denkbar und vorteilhaft.

Die Manschette 10 umfasst außerdem eine in einen Blasenbereich aufgenommene Blase sowie einen Druckschlauch (in Figur 1a und 1b jeweils nicht dargestellt), welche nachfolgend anhand der Figuren 2a und 2b näher beschrieben werden.

In Figur 2a ist erkennbar und dargestellt, dass die Positionierhilfe 13 einen ersten Flügel 23 und einen zweiten Flügel 24 umfasst, wobei die Aussparung 14 in Längsrichtung 1 beidseitig durch die Flügel 23, 24 begrenzt ist. In einem zentralen Bereich des ersten Längsabschnitts 7 vorzugsweise in der Flucht der Aussparung 14 ist ein Druckschlauch 38 angeordnet, an dem ein vorzugsweise mindestens zweifarbiger Informationsträger 39 in Form eines kleinen Fähnchens angebracht ist. Der Druckschlauch 38 ist mit der Blase 16 fluidleitend verbunden, so dass vermittels eines nicht dargestellten Gerätes, ein Fluid über den Druckschlauch 38 der Blase 16 zugeführt werden kann, wodurch die Blase expandierbar ist. Das nicht dargestellte Gerät umfasst vorzugsweise eine Pumpvorrichtung, welche das Fluid in den Druckschlauch zwingt. Das Gerät umfasst in ans sich bekannter Weise vorzugsweise außerdem Anzeigemittel wie ein Display, an dem ein ermittelter Blutdruck angezeigt werden kann und vorzugsweise elektronische Steuermittel, welche den Messvorgang der Blutdruckmessung steuern.

Besonders vorteilhaft ist es, wenn die Positionierhilfe 13 für den Fall, dass die Positionierhilfe sich weiter in Querrichtung nach außen, also nach unten (im angelegten Zustand in Richtung auf die Ellenbeuge), als ein Bereich außerhalb der Positionierhilfe erstreckt, so dass die Manschette im Bereich der Positionierhilfe eine höhere Breite B1 aufweist als ein der Positionierhilfe 13 in Längsrichtung 1 benachbarter Bereich des ersten Längsabschnitts 7 der Manschette 10, in Längsrichtung 1 eine Länge 45 von 10 cm bis 25 cm, noch mehr bevorzugt von 14 cm bis 18 cm aufweist. Die Länge 45, also die maximale Erstreckung der Positionierhilfe in der Längsrichtung 1, wird dabei wie in Figur 2a verdeutlicht im Rahmen der vorliegenden Erfindung solchenfalls dort gemessen, wo die Aussparung 14 sich in Querrichtung 2 am weitesten nach innen in den ersten Längsabschnitt 7 hinein erstreckt.

Alle im Zusammenhang mit der vorliegenden Erfindung vor- und nachstehend beschriebenen Abmessungen wie Abstände, Längen oder Breiten werden an der flachgelegten Manschette vermessen, wobei die Blase noch unbefüllt, also noch nicht expandiert ist.

Die Breite 46 der Aussparung 14 beträgt vorteilhaft wenigstens 2 cm und höchstens 7 cm, noch vorteilhafter wenigstens 3 cm und höchstens 5 cm. Die Breite 46 der Aussparung wird im Rahmen der vorliegenden Erfindung als der Abstand zwischen zwei weiteren gedachten, in der Längsrichtung verlaufenden Linien 51 und 52 verstanden. Hierbei verläuft eine gedachte Aussparungslinie 51 dort, wo die Aussparung 14 sich in Querrichtung 2 am weitesten nach innen in den ersten Längsabschnitt 7 hinein erstreckt. Eine gedachte Flügellinie 52 verläuft durch das distale Ende der Flügel 23, 24, also dort, wo die Flügel sich am weitesten nach außen erstrecken. Sollte sich einer der Flügel weiter nach außen erstrecken als der andere, so wird die gedachte Flügellinie an das distale Ende desjenigen Flügels angelegt, der sich weiter nach außen erstreckt.

Figur 2b und Figur 2a, zeigen überdies, dass die Manschette 10 zwei aneinander gefügte Materiallagen 63 und 64 umfasst, wobei die innere Materiallage 63 die Innenseite 3 und die äußere Materiallage 64 die Außenseite 4 der Manschette 10 bilden. Die inneren und äußeren Materiallagen 63,64 können textile Stoffe, Folien, Nonwoven oder andere flexible, anschmiegsame Materialien umfassen und ihrerseits vorteilhaft ein- oder mehrschichtig ausgebildet sein. Zwischen die innere und äußere Materiallage 63, 64 ist in einem Blasenbereich 15 eine Blase 16 aufgenommen.

Der Blasenbereich 15 ist im dargestellten Fall dadurch gebildet, dass entlang einer vorzugsweise in etwa rechteckförmigen Kontur die beiden Materiallagen 63, 64 durch eine oder mehrere Nähte oder andere Fügeverfahren wie insbesondere Thermoschweißen oder Verkleben unter Ausbildung der Blasenbereichsränder miteinander verbunden sind.

Dargestellt sind in Figur 2b eine untere Naht 57 und eine obere Naht 58. Die untere Naht 57 bildet blasenseitig einen unteren Blasenbereichsrand 17. Die obere Naht 58 bildet blasenseitig einen oberen Blasenbereichsrand. Innerhalb des Blasenbereich 15 verbleiben die beiden Materiallagen 63, 64 unverbunden, so dass ein Hohlraum (der Blasenbereich) gebildet ist, in den die Blase 16 zwischen den beiden Materiallagen 63, 64 aufgenommen ist.

Die Aussparung 14 ist in Querrichtung 2 mit einem ersten Abstand 18 vom unteren Blasenbereichsrand 17 beabstandet, und zwar vorzugsweise um mindestens 1,5 cm, vorzugsweise um 1,5 bis 5,5 cm, weiter vorzugsweise um 2,5 bis 4,0 cm und insbesondere um höchsten 3,0 cm. Der erste Abstand 18 ist hierbei stets der Abstand (also die kürzeste Entfernung) zwischen der gedachten Aussparungslinle 51 und einer weiteren in der Längsrichtung 1 verlaufenden gedachten Blasenbereichslinie 53, welche dort verläuft, wo sich der untere Blasenbereichsrand 17 in Querrichtung 2 am weitesten nach außen, also in Richtung auf die Positionierhilfe 13 erstreckt.

Vorzugsweise beträgt die Länge L1 des ersten Längsabschnitts 7, also dessen maximale Erstreckung in der Längsrichtung 10 cm bis 40 cm, insbesondere 20 cm bis 30 cm, weiter insbesondere 22 cm bis 25 cm. Die Länge L1 des ersten Längsabschnitts wird solchenfalls ermittelt als der Abstand von einem ersten Manschettenquerrand 70 bis zu einem endabschnittnahen Blasenberelchsquerrand 72. Die Länge des verbleibenden Endabschnitts 6 beträgt vorzugsweise 10 cm bis 35 cm, insbesondere 24 cm bis 28 cm. Die Gesamtlänge der Manschette 10 resultiert mithin aus der Summe der Längen des ersten Längsabschnitts 7 und des Endabschnitts 6.

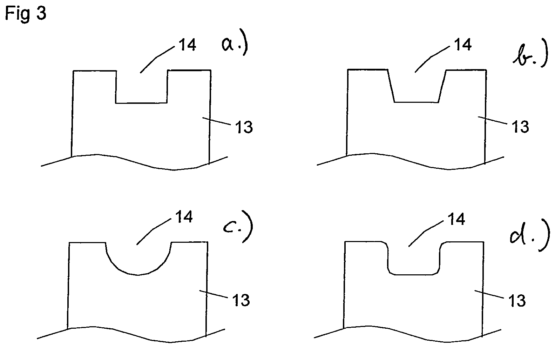

Figuren 3a-d veranschaulichen verschiedene bevorzugte Ausführungen der Positionierhilfe 13. Die Aussparung 14 weist vorzugsweise eine im Wesentlichen rechteckige (Figur 3a) oder trapezförmige (Figur 3b) Kontur auf. Auch eine im Wesentlichen bogen- (Figur 3c) oder kurvenförmige Kontur (Figur 3d) ist vorteilhaft, Unterschiedliche Varianten der Aussparung sind dadurch besonders vorteilhaft, dass abhängig von der Form und Größe der Ellenbeuge eines Benutzers eine Positionierhilfe 13 gewählt werden kann, die im angelegten Zustand der Manschette möglichst passgenau in der Ellenbeuge des Benutzers zu liegen kommt. Andere, nicht dargestellte Konturen der Aussparung, insbesondere Kombinationen aus kurvenförmigen und geradlinigen Konturabschnitten sind ebenfalls denkbar und vorteilhaft.

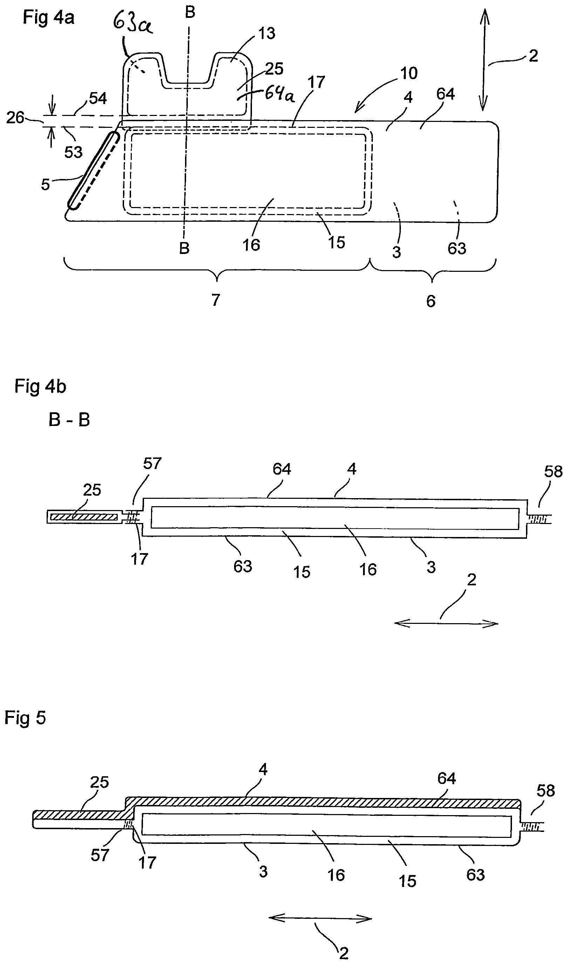

Die Figuren 4a, 4b und 5 zeigen Varianten der vorliegenden Erfindung mit einer ein Versteifungselement aufweisenden Positionierhilfe.

In der Ausführungsform der Figuren 4a, 4b umfasst die Positionierhilfe 13 ein Versteifungselement 25, welches sandwichartig zwischen den beiden die Innenseite 3 und die Außenseite 4 der die Positionierhilfe 13 bildenden inneren 63a und äußeren Materiallage 64b angeordnet ist. Dabei zeigt Figur 4b einen Querschnitt durch Figur 4a entlang der in Figur 4a gezeigten Linie B-B. Das Versteifungselement 25 versteift die ansonsten flexible innere 63a und äußere Materiallage 64a. Hierdurch wird die Positionierhilfe 13, insbesondere vorteilhaft die Form der Flügel 23, 24 gegen Verknicken und Verbiegen stabilisiert. Dem Benutzer wird auf diese Weise beim Anlegen die korrekte Positionierung der Manschette 10 erleichtert.

Die Positionierhilfe 13 ist in der dargestellten Ausführungsform als ein separater Materialabschnitt mittels einer unteren Naht 57 mit dem die Blase 16 umfassenden Teil der Manschette 10 verbunden, wobei die Positionierhilfe im Bereich der Naht 57 zwischen der inneren 63 und äußere Materiallage 64 der Manschette 10 angeordnet ist. Die untere Naht 57 bildet blasenseitig zugleich die untere Begrenzung des Blasenbereichs 15, mithin den unteren Blasenbereichsrand 17. Die obere Begrenzung des Blasenbereichs 15 wird dann durch die obere Naht 58 gebildet. Das Versteifungselement 25 ist im vorliegenden Fall als eine dünne, entsprechend konturierte Hartkunststoffplatte ausgeführt und erstreckt sich in Querrichtung 2 bis nahe an die untere Naht 57.

Denkbar und vorteilhaft wäre es in einer anderen Ausführungsform der Manschette (nicht dargestellt), wenn der Blasenbereich als eine separate, zwischen die die Innenseite und die Außenseite bildenden Materiallagen aufgenommene Komponente, vorzugsweise in Form einer separaten die Blase vorzugsweise allseitig umgebenden Hülle oder Tasche vorgesehen ist, die solchenfalls den Blasenbereich bildet.

Es ist vorteilhaft, wenn das Versteifungselement 25 einen zweiten Abstand 26 von dem unteren Blasenbereichsrand 17 aufweist. Vorteilhaft beträgt der zweite Abstand 26 höchstens 6,0 cm, weiter vorzugsweise höchsten 5,0 cm, insbesondere zwischen 0,25 cm und 4,0 cm, weiter insbesondere zwischen 0,8 cm und 1,5 cm. Wie in Figur 4a veranschaulicht wird unter dem zweiten Abstand 26 die kürzeste Entfernung zwischen der gedachten Blasenbereichslinie 53 und einer gedachten Versteifungselementlinie 54 verstanden. Hierbei verläuft die gedachte Versteifungselementlinie 54 in der Längsrichtung 1 dort, wo das Versteifungselement 25 seine in Querrichtung 2 weiteste Erstreckung nach innen, in den ersten Längsabschnitt 7 hinein aufweist.

Nach einer weiteren bevorzugten Ausführungsform ist das Versteifungselement 25 auf der (zumindest bereichsweise) die Außenseite 4 der Manschette bildenden Materiallage 64 angefügt (Figur 5). Die Positionierhilfe 13 ist im dargestellten Fall der Figur 5 wie bereits im Zusammenhang der Figur 1a beschrieben durch eine Weitererstreckung des ersten Längsabschnitts 7 nach außen gebildet. Das Versteifungselement 25 erstreckt sich in dieser Ausführungsform über die untere Naht 57 und über den unteren Blasenbereichsrand 17 hinaus und bis nahe an die obere Naht 58 und Überfängt somit zumindest bereichsweise den Blasenbereich 15 und die Blase 16 und bildet somit zumindest bereichsweise die Außenseite 4 der Manschette.

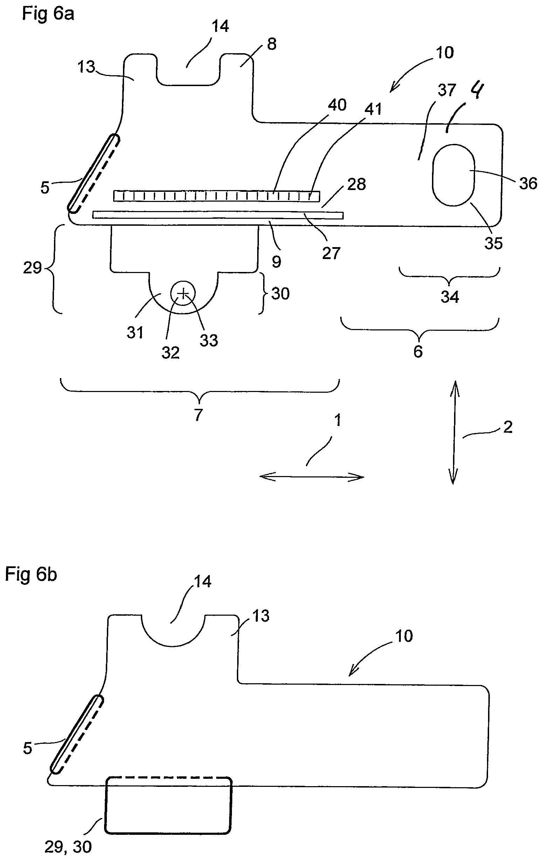

In Figur 6a ist eine weitere Variante der erfindungsgemäßen bandförmigen Manschette 10 dargestellt (auch in Figur 6a und den nachfolgenden Figuren 6b-6d sind jeweils nicht alle Komponenten der Manschette dargestellt, um die Figuren nicht zu überfrachten).

Die Manschette 10 umfasst ein Schlaufenelement 5 und an einem ersten unteren Randbereich 8 eines ersten Längsabschnitts 7 eine Positionierhilfe 13 mit einer Aussparung 14. Die Manschette umfasst außerdem (nicht dargestellt) einen Blasenbereich mit einer expandierbaren Blase, Haftbereiche auf der Außenseite 4 der Manschette zum Fixieren der Manschette um den Oberarm eines Benutzers und gegebenenfalls eine oder mehrere der weiteren Komponenten, vorzugsweise wie vorstehend im Zusammenhang mit den Figuren 1-5 beschrieben.

Weiterhin weist die Manschette 10 zumindest an einem ersten oberen Randbereich 9 einen sich in der Längsrichtung 1 erstreckenden Orientierungsbereich 27 auf (Figur 6a), der sich durch Farbe, Material oder Steifigkeit von einem in Querrichtung 2 in Richtung der Positionierhilfe 13 an den Orientierungsbereich 27 angrenzenden Bereich 28 der Manschette unterscheidet. Bevorzugt handelt es sich bei dem Orientierungsbereich 27 um einen farblich abgesetzten Streifen, insbesondere einen roten oder orangfarbenen Streifen, der auf der Außenseite 4 der Manschette angebracht ist und entweder durch die Materiallage 64 der Außenseite 4 selbst gebildet wird oder auf dieser befestigt ist. Hierdurch wird es einem Benutzer der Manschette erleichtert, den ersten oberen Randbereich 9 zu erkennen und die bandförmige Manschette richtig herum um den Oberarm anzulegen. Somit wird die Gefahr einer nicht korrekten Orientierung der Manschette verringert und die Genauigkeit einer Blutdruckmessung damit erhöht.

Weiterhin umfasst die dargestellte bandförmige Manschette zumindest an dem ersten Längsabschnitt 7 einen sich in der Längsrichtung 1 erstreckenden Ablesebereich 40 mit Ablesemarkierungen 41 (Figur 6a). Die Ablesemarkierungen 41 werden vorzugsweise durch Linien, Punkte, Symbole, Farbmuster, Zahlen, Buchstabenfolgen oder Kombinationen aus den genannten Markierungen gebildet. Bevorzugt ist es, wenn die Ablesemarkierungen Zahlen aufweisen, die in Form einer Skala auf dem Ablesebereich 40 angeordnet sind und sich in ihrer Farbe vom Hintergrund des Ablesebereichs 40 abheben. Hierdurch kann der Benutzer der Manschette zum Messen des Blutdrucks den Umfang der angelegten Manschette ablesen und erinnern oder aufzeichnen, um bei der nächsten Messung des Blutdrucks wieder den gleichen Umfang der Manschette einstellen zu können. Auf diese Weise wird die Reproduzierbarkeit von Blutdruckmessungen, insbesondere durch nicht geschulte Benutzer, erhöht.

Besonders bevorzugt ist es außerdem, wenn der Ablesebereich 40 mit dem Orientierungsbereich 27 derart kombiniert ist, dass der Ablesebereich 40 Zahlen umfasst, die auf einem andersfarbigen Streifen in dem ersten oberen Randbereich 9 des ersten Längsabschnitts 7 angeordnet sind, so dass Ablese- und Orientierungsbereich zusammenfallen (nicht dargestellt in Figur 6a).

Des Weiteren umfasst der Endabschnitt 6 vorzugsweise wie dargestellt (Figur 6a) an einem Randbereich 34 des Endabschnitts 6 eine zweite Anlegehilfe 35, die einen zweiten Anfassbereich 36 aufweist. Dieser unterscheidet sich in Farbe, Material oder Steifigkeit von einem Teilbereich 37 des Endabschnitts 6, welcher sich in Längsrichtung 1 an den zweiten Anfassbereich 36 in Richtung auf den ersten Längsabschnitt 7 anschließt. Hierdurch wird es dem Benutzer erleichtert, so am zweiten Anfassbereich 36 des Endabschnitts 6 zu ziehen, dass der korrekte Umfang der Manschette auf dem Oberarm des Benutzers eingestellt wird.

Der erste obere Randbereich 9 umfasst außerdem vorzugsweise eine erste Anlegehilfe 29, die ein, insbesondere daran angefügtes, erstes Anfassmittel 30 aufweist oder daraus gebildet ist. Diese so konfigurierte erste Anlegehilfe 29 stellt dabei einen an sich unabhängigen Erfindungsgedanken dar. Die erste Anlegehilfe 29 kann somit vorzugsweise auch bei an sich üblichen Manschetten zum Messen des Blutdrucks vorgesehen sein, also insbesondere solchen Manschetten, die keine Positionierhilfe 13 mit Aussparung 14 aufweisen.

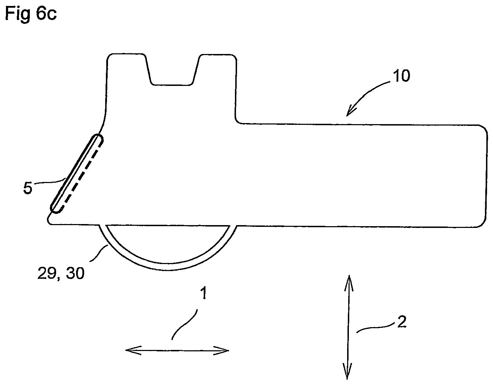

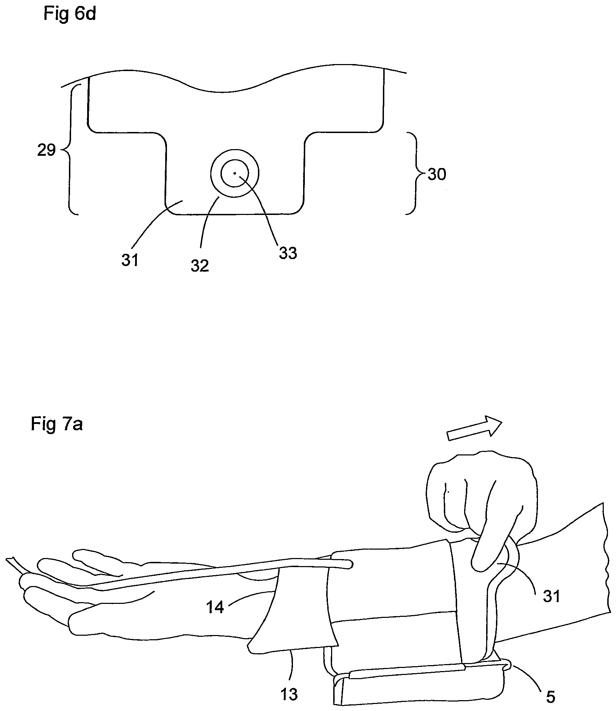

Das erste Anfassmittel 30 umfasst bevorzugt eine Lasche (Figuren 6a, 6d), einen Bügel (Figur 6b), einen Griff, einen Henkel (Figur 6c) oder einen Haken.

Ist das erste Anfassmittel 30 als ein Bügel oder ein Griff oder ein Haken ausgeführt, so umfasst dieser bevorzugt ein Metall oder einem Kunststoff mit hoher Biegesteifigkeit. Die Befestigung an dem ersten oberen Randbereich 9 erfolgt solchenfalls bevorzugt durch eine oder mehrere Taschen, die in ähnlicher Weise wie im Zusammenhang mit dem Schlaufenelement 5 beschrieben, das erste Anfassmittel 30 mit der bandförmigen Manschette 10 verbinden.

Bei einer Ausführung des ersten Anfassmittels 30 als Lasche (Figur 6a, 6d) ist diese bevorzugt so gestaltet, dass sie eine geringe Biegesteifigkeit aufweist. Dabei beinhaltet das erste Anfassmittel 30 vorzugsweise einen Greifbereich 31. Innerhalb des Greifbereichs 31 ist vorzugsweise ein Markierungsbereich 32 vorgesehen, wobei der Markierungsbereich 32 vorzugsweise eine vom Greifbereich 31 durch Farbe, Material oder Steifigkeit, insbesondere visuell unterscheidbare Greifmarkierung 33 aufweist. Hierdurch wird des dem Benutzer erleichtert, einen zum Anfassen besonders geeigneten Bereich zu erkennen.

In einer bevorzugten Ausführungsform ist diese Greifmarkierung 33 ein aufgedrucktes Symbol, wie beispielsweise ein Kreuz (Figur 6a), das eine andere Farbe als der Greifbereich 31 im Übrigen aufweist. In einer weiteren Ausführungsform ist diese Greifmarkierung 33 ein Kreis oder weist mehrere konzentrische Kreise (Figur 6d) mit einer anderen Farbe als der Greifbereich 31 auf. Hierdurch wird es dem Benutzer erleichtert, an einer besonders vorteilhaften Stelle der Manschette 10, nämlich der Greifmarkierung, anzufassen und die Manschette durch Ziehen in Richtung des Schultergelenks korrekt auf dem Oberarm zu positionieren. Der Benutzer kann hierbei die Greifmarkierung 33 zwischen Daumen und Zeigefinger angreifen, um Zug auszuüben.

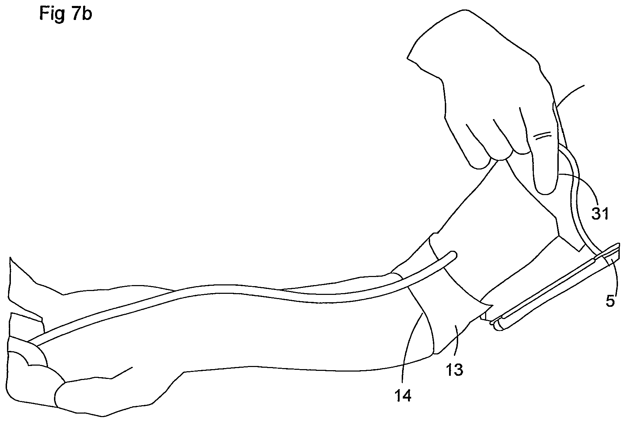

Figuren 7a und 7b veranschaulichen den Positioniervorgang einer bevorzugten Ausführungsform mit erster Anlegehilfe. Der Greifbereich 31 des Anfassmittels der Anlegehilfe wird an der im Markierungsbereich angeordneten Greifmarkierung vom Benutzer zwischen zwei Fingern gegriffen. Danach zieht der Benutzer in Richtung des Schultergelenks (Figur 7a), bis die Manschette mit der Aussparung 14 der Positionierhilfe 13 in der Ellenbeuge des Benutzers anliegt (Figur 7b).

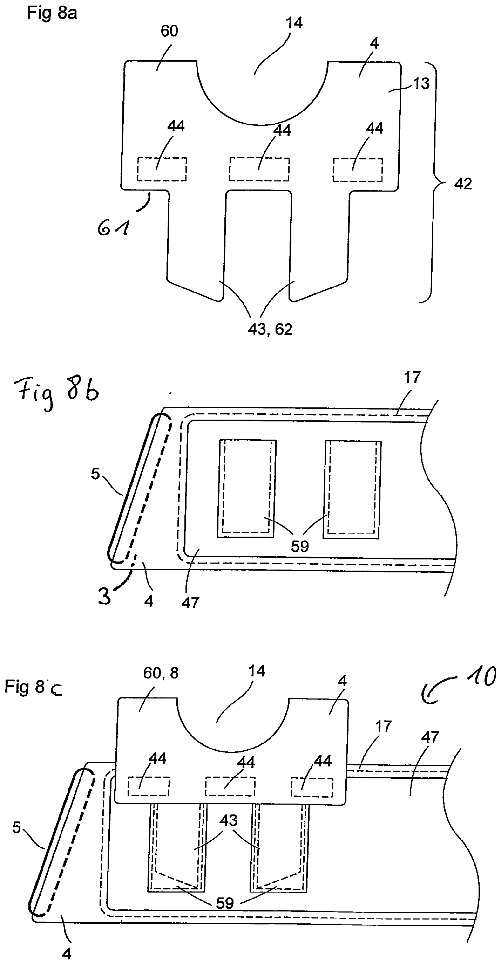

Figur 8a zeigt eine separate, die spätere Positionierhilfe 13 aufweisende Positioniervorrichtung 42 zum lösbaren Festlegen an einer bandförmige Manschette. Hierzu weist die Positioniervorrichtung 42 an ihrem äußeren Längsrand 60 eine Positionierhilfe 13, umfassend eine Aussparung 14, auf. An einem inneren Längsrand 61 weist die Positioniervorrichtung 42 bevorzugt mindestens zwei Vorsprünge 62 auf, welche erste Befestigungsmittel 43 bilden.

Die bandförmige Manschette 10 (Figur 8b) weist solchenfalls in dem ersten Längsabschnitt 7, welcher das Schlaufenelement 5 umfasst, vorzugsweise Taschen 59 auf, wobei die Taschen 59 auf ihrer dem ersten unteren Randbereich 8 der Manschette zugewandten Seite offen sind. Die Taschen können wie dargestellt auf die Außenseite 4 der Manschette 10 aufgesetzt, beispielsweise aufgenäht sein; denkbar und vorteilhaft wäre auch, die Taschen sandwichartig zwischen die innere und die äußere Materiallagen anzuordnen, welche die Innenseite 3 und die Außenseite 4 der Manschette 10 bilden. Mit Bezugszeichen 17 ist der untere Blasenbereichsrand gekennzeichnet. Die Blase ist nicht dargestellt.

Die die Befestigungsmittel 43 bildenden Vorsprünge 62 sind dann zur Fixierung der Positioniervorrichtung 42 an der Manschette 10 in die dafür vorgesehenen Taschen 59 einschiebbar. Vorteilhafterweise umfassen die Vorsprünge 62 und die Taschen 59 miteinander kooperierende Rastmittel, wie Einrastnasen und korrespondierende Haltemittel, so dass die Vorsprünge in den Taschen 59 in einer vorgegebenen Stellung lösbar gehalten sind.

Denkbar und vorteilhaft wäre auch, das lösbare Festlegen der Positioniervorrichtung 42 an der Manschette alternativ zu den ersten Befestigungsmitteln oder wie dargestellt in Ergänzung dazu durch auf der Innenseite der Positioniervorrichtung 42 vorzusehende Klettbereiche 44 der Positioniervorrichtung, vorzugsweise in Form von Kletthaken, zu ermöglichen, welche mit einer schlaufenbildende Zone 47, vorzugsweise einem Flauschmaterial, auf der Außenseite 4 des ersten Längsabschnitts 7 in Eingriff bringbar sind. Die schlaufenbildende Zone 47 ist überdies vorzugsweise zum Schließen der Manschette mit einem in Figuren 8b, 8c nicht dargestellten Haftbereich des Endabschnitts der Manschette in Eingriff bringbar.

In Figur 8c ist die an die Manschette 10 lösbar angefügte Positioniervorrichtung 42 dargestellt. Die erste Befestigungsmittel 43 bildenden Vorsprünge 62 sind in die Taschen 59 eingeschoben. Außerdem haften die Kletthaken umfassenden Klettbereiche der Positioniervorrichtung 44 an der Außenseite 4 der Manschette, welche dort eine entsprechende schlaufenbildende Zone 47 aufweisen, die aus einem Flauschmaterial gebildet sind. Im Ergebnis ist eine erfindungsgemäße bandförmige Manschette 10 bereitgestellt, welche an einem ersten Längsabschnitt 7 an einem ersten unteren Randbereich 8 eine Positionierhilfe 13 aufweist, wobei die Positionierhilfe 13 eine Aussparung 14 umfasst, so dass die Manschette passgenau an die Ellenbeuge anlegbar ist und die Gefahr des Verrutschens in Umfangsrichtung der angelegten Manschette reduziert ist.

Mit der lösbar festlegbaren Positioniervorrichtung 42 hat der Benutzer die Wahl, die Manschette gegebenenfalls in wie im Stand der Technik bekannter Weise auch ohne Positionierhilfe zu verwenden.

## Patentansprüche

1. Bandförmige Manschette (10) für das Messen des Blutdrucks mit einer Längsrichtung (1) und einer Querrichtung (2) und mit einer Innenseite (3) und einer Außenseite (4), wobei die bandförmige Manschette (10) ein Schlaufenelement (5) umfasst, und mit einem Endabschnitt (6), der durch das Schlaufenelement (5) hindurch führbar ist, wobei die bandförmige Manschette (10) einen ersten Längsabschnitt (7) aufweist, der das Schlaufenelement (5) umfasst, wobei der erste Längsabschnitt (7) einen ersten unteren Randbereich (8) sowie einen ersten oberen Randbereich (9) aufweist, und wobei die bandförmige Manschette (10) einen Blasenbereich (15) umfasst, wobei der Blasenbereich (15) eine Blase (16) aufweist, und der erste Längsabschnitt (7) an einem ersten unteren Randbereich (8) eine Positionierhilfe (13) aufweist, wobei die Positionierhilfe (13) eine Aussparung (14) umfasst, die Positionierhilfe (13) einen ersten Flügel (23) und einen zweiten Flügel (24) umfasst und die Aussparung (14) in Längsrichtung (1) beidseitig durch die Flügel (23, 24) begrenzt ist und der Blasenbereich (15) einen unteren Blasenbereichsrand (17) aufweist und wobei die Aussparung (14) in Querrichtung (2) einen ersten Abstand (18) von 1,5 bis 5,5 cm von dem unteren Blasenbereichsrand (17) aufweist.

2. Bandförmige Manschette (10) für das Messen des Blutdrucks nach Anspruch 1, wobei die Aussparung (14) eine im Wesentlichen rechteckige oder trapezförmige Kontur aufweist.

3. Bandförmige Manschette (10) für das Messen des Blutdrucks nach einem der vorhergehenden Ansprüche, wobei die Aussparung (14) eine im Wesentlichen bogen- oder kurvenförmige Kontur aufweist.

4. Bandförmige Manschette (10) für das Messen des Blutdrucks nach Anspruch 3, wobei die Positionierhilfe, insbesondere der erste und/oder der zweite Flügel (23,24) ein Versteifungselement (25) aufweist oder davon gebildet wird.

5. Bandförmige Manschette (10) für das Messen des Blutdrucks nach Anspruch 4, wobei das Versteifungselement (25) auf der die Innenseite (3) oder der die Außenseite (4) oder zwischen der die Innenseite (3) der Positionierhilfe bildenden Materiallage (63, 63a) und der die Außenseite (4) der Positionierhilfe bildenden Materiallage (64, 64b) angeordnet ist.

6. Bandförmige Manschette (10) für das Messen des Blutdrucks nach einem der vorhergehenden Ansprüche 4 bis 5, wobei das Versteifungselement (25) in Querrichtung (2) einen zweiten Abstand (26) von höchstens 6,0 cm, vorzugsweise von höchsten 5,0 cm, insbesondere zwischen 0,25 cm und 4,0 cm, und weiter insbesondere zwischen 0,8 cm und 1,5 cm von dem unteren Blasenbereichsrand (17)aufweist oder sich in Querrichtung (2) über den unteren Blasenbereichsrand (17) hinaus erstreckt.

7. Bandförmige Manschette (10) für das Messen des Blutdrucks nach einem der vorhergehenden Ansprüche, wobei die Manschette (10) in Längsrichtung (1) zumindest in einem ersten oberen Randbereich (9) einen Orientierungsbereich (27) aufweist, der sich durch Farbe, Material oder Steifigkeit von einem in Querrichtung (2) an den Orientierungsbereich (27) angrenzenden Bereich (28) der Manschette (10) unterscheidet.

8. Bandförmige Manschette (10) für das Messen des Blutdrucks nach einem der vorhergehenden Ansprüche, wobei der erste obere Randbereich (9) eine erste Anlegehilfe (29) mit einem ersten Anfassmittel (30) aufweist.

9. Bandförmige Manschette (10) für das Messen des Blutdrucks nach Anspruch 8, wobei das erste Anfassmittel (30) einen Greifbereich (31) und einen Markierungsbereich (32) umfasst, wobei der Markierungsbereich (32) eine vom Greifbereich (31) durch Farbe, Material oder Steifigkeit, insbesondere visuell unterscheidbare Greifmarkierung (33) aufweist.

10. Bandförmige Manschette (10) für das Messen des Blutdrucks nach einem der vorhergehenden Ansprüche, wobei der Endabschnitt (6) an einem Randbereich (34) eine zweite Anlegehilfe (35) umfasst, wobei die zweite Anlegehilfe (35) einen zweiten Anfassbereich (36) aufweist, und wobei sich der zweite Anfassbereich (36) in Farbe, Material oder Steifigkeit unterscheidet von einem Teilbereich (37) des Endabschnitts (6), welches sich in Längsrichtung (1) an den zweiten Anfassbereich (36) anschließt.

11. Bandförmige Manschette (10) für das Messen des Blutdrucks nach einem der vorhergehenden Ansprüche, wobei die bandförmige Manschette (10) einen Druckschlauch (38) zum Einleiten eines Fluids in die Blase (16) umfasst, wobei an dem Druckschlauch (38) vorzugsweise ein mindestens zweifarbiger Informationsträger (39) angebracht ist.

12. Bandförmige Manschette (10) für das Messen des Blutdrucks nach einem der vorhergehenden Ansprüche, wobei die Manschette (10) zumindest an einem ersten Längsabschnitt (7) einen sich in der Längsrichtung (1) erstreckenden Ablesebereich (40) mit Ablesemarkierungen (41) aufweist.

13. Bandförmige Manschette (10) für das Messen des Blutdrucks nach einem der vorhergehenden Ansprüche, wobei die Positionierhilfe (13) an einer von der Manschette (10) ablösbaren und wieder festlegbaren Positioniervorrichtung (42) angeordnet ist.

14. Bandförmige Manschette (10) für das Messen des Blutdrucks nach einem der vorhergehenden Ansprüche, wobei die Innenseite (3) des ersten Längsabschnitts (7) ein Material umfasst oder davon gebildet ist, welches der Innenseite (3) des ersten Längsabschnitts (7) auf der Haut eines Benutzers eine höhere Haftung als eine Außenseite (4) des ersten Längsabschnitts (7) verleiht.

## Claims

1. Strip-shaped cuff (10) for measuring blood pressure, said cuff having a longitudinal direction (1) and a transverse direction (2) and having an inner side (3) and an outer side (4), wherein the strip-shaped cuff (10) comprises a loop element (5), and having an end portion (6), which can be passed through the loop element (5), wherein the strip-shaped cuff (10) has a first longitudinal portion (7), which comprises the loop element (5), wherein the first longitudinal portion (7) has a first lower edge region (8) and also a first upper edge region (9), and wherein the strip-shaped cuff (10) comprises a bladder region (15), wherein the bladder region (15) comprises a bladder (16), and the first longitudinal portion (7) comprises, at a first lower edge region (8), a positioning aid (13), wherein the positioning aid (13) comprises a recess (14), the positioning aid (13) comprises a first wing (23) and a second wing (24), and the recess (14) is defined on either side in the longitudinal direction (1) by the wings (23, 24), and the bladder region (15) comprises a lower bladder region edge (17), and wherein the recess (14) in the transverse direction (2) has a first spacing (18) of 1.5 to 5.5 cm from the lower bladder region edge (17).

2. Strip-shaped cuff (10) for measuring blood pressure according to Claim 1, wherein the recess (14) has a substantially rectangular or trapezoidal contour.

3. Strip-shaped cuff (10) for measuring blood pressure according to one of the preceding claims, wherein the recess (14) has a substantially arcuate or curved contour.

4. Strip-shaped cuff (10) for measuring blood pressure according to Claim 3, wherein the positioning aid, in particular the first and/or the second wing (23, 24), comprises a reinforcement element (25) or is formed thereby.

5. Strip-shaped cuff (10) for measuring blood pressure according to Claim 4, wherein the reinforcement element (25) is arranged on the material layer (63, 63a) forming the inner side (3) or on the material layer (63, 63a) forming the outer side (4) or between the material layer (63, 63a) forming the inner side (3) of the positioning aid and the material layer (64, 64b) forming the outer side (4) of the positioning aid.

6. Strip-shaped cuff (10) for measuring blood pressure according to one of preceding Claims 4 to 5, wherein the reinforcement element (25) in the transverse direction (2) has a second spacing (26) of at most of 6.0 cm, preferably of at most 5.0 cm, in particular between 0.25 cm and 4.0 cm, and more particularly between 0.8 cm and 1.5 cm, from the lower bladder region edge (17) or extends beyond the lower bladder region edge (17) in the transverse direction (2) .

7. Strip-shaped cuff (10) for measuring blood pressure according to one of the preceding claims, wherein the cuff (10) in the longitudinal direction (1), at least in a first upper edge region (9), comprises an orientation region (27), which differs in terms of color, material or rigidity from a region (28) of the cuff (10) adjacent to the orientation region (27) in the transverse direction (2).

8. Strip-shaped cuff (10) for measuring blood pressure according to one of the preceding claims, wherein the first upper edge region (9) comprises a first fitting aid (29) with a first tactile means (30).

9. Strip-shaped cuff (10) for measuring blood pressure according to Claim 8, wherein the first tactile means (30) comprises a grip region (31) and a marking region (32), wherein the marking region (32) comprises a grip marking (33) that is in particular visually distinguishable from the grip region (31) in terms of color, material or rigidity.

10. Strip-shaped cuff (10) for measuring blood pressure according to one of the preceding claims, wherein the end portion (6) at an edge region (34) comprises a second fitting aid (35), wherein the second fitting aid (35) comprises a second tactile region (36), and wherein the second tactile region (36) differs in terms of color, material or rigidity from a sub-region (37) of the end portion (6) adjoining the second tactile region (36) in the longitudinal direction (1).

11. Strip-shaped cuff (10) for measuring blood pressure according to one of the preceding claims, wherein the strip-shaped cuff (10) comprises a pressure tubing (38) for introducing a fluid into the bladder (16), wherein an information carrier (39), which is preferably at least bi-colored, is attached to the pressure tubing (38).

12. Strip-shaped cuff (10) for measuring blood pressure according to one of the preceding claims, wherein the cuff (10) comprises, at least on a first longitudinal portion (7), a read-off region (40) extending in the longitudinal direction (1) and comprising read-off markings (41).

13. Strip-shaped cuff (10) for measuring blood pressure according to one of the preceding claims, wherein the positioning aid (13) is arranged on a positioning device (42) that can be detached from the cuff (10) and reattached thereto.

14. Strip-shaped cuff (10) for measuring blood pressure according to one of the preceding claims, wherein the inner side (3) of the first longitudinal portion (7) comprises a material or is formed thereby, said material lending the inner side (3) of the first longitudinal portion (7) a greater level of adhesion on a user's skin than an outer side (4) of the first longitudinal portion (7).

## Revendications

1. Brassard en forme de bande (10) pour la mesure de la pression sanguine avec une direction longitudinale (1) et une direction transversale (2) et avec un côté intérieur (3) et un côté extérieur (4), le brassard en forme de bande (10) comprenant un élément de boucle (5), et avec une partie d'extrémité (6), qui peut être menée à travers l'élément de boucle (5), le brassard en forme de bande (10) présentant une première partie longitudinale (7), qui comprend l'élément de boucle (5), la première partie longitudinale (7) présentant une première zone de bord inférieure (8) ainsi qu'une première zone de bord supérieure (9), et le brassard en forme de bande (10) comprenant une zone de bulle (15), la zone de bulle (15) présentant une bulle (16), et la première partie longitudinale (7) présentant un accessoire de positionnement (13) sur une première zone de bord inférieure (8), l'accessoire de positionnement (13) présentant une découpe (14), l'accessoire de positionnement (13) comprenant une première aile (23) et une deuxième aile (24) et la découpe (14) étant limitée en direction longitudinale (1) de part et d'autre par les ailes (23, 24) et la zone de bulle (15) présentant un bord de zone de bulle inférieur (17) et la découpe (14) présentant dans la direction transversale (2) une première distance (18) de 1,5 à 5,5 cm du bord de zone de bulle inférieur (17).

2. Brassard en forme de bande (10) pour la mesure de la pression sanguine selon la revendication 1, dans lequel la découpe (14) présente un contour essentiellement rectangulaire ou trapézoïdal.

3. Brassard en forme de bande (10) pour la mesure de la pression sanguine selon l'une quelconque des revendications précédentes, dans lequel la découpe (14) présente un contour essentiellement en forme d'arc ou de courbe.

4. Brassard en forme de bande (10) pour la mesure de la pression sanguine selon la revendication 3, dans lequel l'accessoire de positionnement, en particulier la première et/ou la deuxième aile (23, 24), présente un élément de renforcement (25) ou en est formé.

5. Brassard en forme de bande (10) pour la mesure de la pression sanguine selon la revendication 4, dans lequel l'élément de renforcement (25) est disposé sur la couche de matière (63, 63a) formant le côté intérieur (3) ou sur la couche de matière (63, 63a) formant le côté extérieur (4) ou entre la couche de matière (63, 63a) formant le côté intérieur (3) de l'accessoire de positionnement et la couche de matière (64, 64b) formant le côté extérieur (4) de l'accessoire de positionnement.

6. Brassard en forme de bande (10) pour la mesure de la pression sanguine selon l'une quelconque des revendications précédentes 4 à 5, dans lequel l'élément de renforcement (25) présente dans la direction transversale (2) une deuxième distance (26) de maximum 6,0 cm, de préférence de maximum 5,0 cm, en particulier comprise entre 0,25 cm et 4,0 cm, et en particulier encore comprise entre 0,8 cm et 1,5 cm du bord de zone de bulle inférieur (17) ou s'étend en direction transversale (2) au-delà du bord de zone de bulle inférieur (17).

7. Brassard en forme de bande (10) pour la mesure de la pression sanguine selon l'une quelconque des revendications précédentes, le brassard (10) présentant dans la direction longitudinale (1) au moins dans une première zone de bord supérieure (9) une zone d'orientation (27), qui se distingue par la couleur, la matière ou la raideur d'une zone (28) du brassard (10) adjacente à la zone d'orientation (27) dans la direction transversale (2).

8. Brassard en forme de bande (10) pour la mesure de la pression sanguine selon l'une quelconque des revendications précédentes, dans lequel la première zone de bord supérieure (9) présente un premier accessoire d'application (29) avec un premier moyen de saisie (30).

9. Brassard en forme de bande (10) pour la mesure de la pression sanguine selon la revendication 8, dans lequel le premier moyen de saisie (30) comprend une zone de prise (31) et une zone de marquage (32), dans lequel la zone de marquage (32) présente un marquage de prise (33) pouvant être distingué de la zone de prise (31) par la couleur, la matière ou la raideur, en particulier visuellement.

10. Brassard en forme de bande (10) pour la mesure de la pression sanguine selon l'une quelconque des revendications précédentes, dans lequel la partie d'extrémité (6) comprend un deuxième accessoire d'application (35) sur une zone de bord (34), dans lequel le deuxième accessoire d'application (35) présente une deuxième zone de saisie (36), et dans lequel la deuxième zone de saisie (36) se distingue par la couleur, la matière ou la raideur, d'une zone partielle (37) de la partie d'extrémité (6), qui se raccorde à la deuxième zone de saisie (36) dans la direction longitudinale (1).

11. Brassard en forme de bande (10) pour la mesure de la pression sanguine selon l'une quelconque des revendications précédentes, le brassard en forme de bande (10) comprenant un tuyau souple sous pression (38) pour l'introduction d'un fluide dans la bulle (16), de préférence un support d'information (39) au moins bicolore étant appliqué sur le tuyau souple sous pression (38) .

12. Brassard en forme de bande (10) pour la mesure de la pression sanguine selon l'une quelconque des revendications précédentes, le brassard (10) présentant au moins sur une première partie longitudinale (7) une zone de lecture (40) s'étendant dans la direction longitudinale (1) avec des marquages lisibles (41).

13. Brassard en forme de bande (10) pour la mesure de la pression sanguine selon l'une quelconque des revendications précédentes, dans lequel l'accessoire de positionnement (13) est disposé sur un dispositif de positionnement (42) détachable du brassard (10) et pouvant y être de nouveau fixé.

14. Brassard en forme de bande (10) pour la mesure de la pression sanguine selon l'une quelconque des revendications précédentes, dans lequel le côté intérieur (3) de la première partie longitudinale (7) comprend une matière ou en est formée, lequel confère au côté intérieur (3) de la première partie longitudinale (7) une meilleure adhérence sur la peau d'un utilisateur qu'un côté extérieur (4) de la première partie longitudinale (7) .
